# EUROPÄISCHE PATENTSCHRIFT

(11) **EP 1 555 986 B1**
(45) Veröffentlichungstag und Bekanntmachung des Hinweises auf die Patenterteilung: **14.03.2007**
(21) Anmeldenummer: 03750643.3
(22) Anmeldetag: 26.09.2003
(51) Int. Cl.: A61K 8/64, A61Q 5/04, A61Q 5/08, A61Q 5/10

(54) **VERWENDUNG VON NATIVEM ZEIN ZUR VERBESSERUNG DES HAARZUSTANDES UND MITTEL**
USE OF NATIVE ZEIN FOR IMPROVING THE CONDITION OF HAIR, AND CORRESPONDING AGENT
UTILISATION DE ZEINE NATIVE AFIN D'AMELIORER L'ETAT DES CHEVEUX ET AGENT CORRESPONDANT

(30) Priorität: 30.10.2002 DE 10250562
(43) Veröffentlichungstag der Anmeldung: 27.07.2005
(73) Patentinhaber: Wella Aktiengesellschaft, 64274 Darmstadt (DE)
(72) Erfinder: KELLER, Walter, 64372 Ober Ramstadt (DE); KISCHKA, Karl-Heinz, 64293 Darmstadt (DE)
(86) Internationale Anmeldenummer: PCT/EP2003/010747
(87) Internationale Veröffentlichungsnummer: WO 2004/039340

(56) Entgegenhaltungen:
- WO-A-03/055456
- US-A- 2 383 990
- DATABASE WPI Section Ch, Week 199142 Derwent Publications Ltd., London, GB; Class D21, AN 1991-307296 XP002267232 & JP 03 206015 A (KATAYAMA KAGAKU KOGYO KENKYUSH), 9. September 1991 (1991-09-09)
- PATENT ABSTRACTS OF JAPAN A
- "International Cosmetic Ingredient Dictionary and Handbook, 9.Aufl. Bd.2" 18. Januar 2002 (2002-01-18), CTFA , WASHINGTON (US) 021644 * Seite 1851 *

## Beschreibung

Gegenstand der vorliegenden Erfindung ist die Verwendung von nativem Zein zur Härtung, Stärkung und Strukturverbesserung (Restrukturierung) von geschädigten menschlichen Haaren.

Die Schädigung von keratinischen Fasern durch Umwelteinflüsse (beispielsweise energiereiche Strahlungen), den physiologischen Status (beispielsweise Alter oder Gesundheit der betreffenden Individuen) oder mechanische und chemische Einwirkungen sind bekannt. Die Folgen sind nachteilige mechanische Eigenschaften der betroffenen Materialien. Derartige Schädigungen der inneren Struktur von keratinischen Fasern zeigen sich zum Beispiel durch Verlust an Härte, Glanz, Stärke, Bruchfestigkeit, Reißfestigkeit oder Bündelzugfestigkeit.

An Keratinfasern, besonders an menschlichen Haaren, machen sich solche Einflüsse insbesondere durch fehlenden Glanz, verminderte Reißkraft und schlechte Kämmbarkeit bemerkbar. Hervorgerufen werden sie durch Alterungsprozesse, die vor allem physiologisch bedingt sind oder durch physikalische (Bewitterung), mechanische (Kämmen, Bürsten) und chemische Einflüsse induziert werden. Bei längeren Haaren machen sich diese Einflüsse insbesondere in den Haarspitzen bemerkbar. Zu den chemischen Einflüssen gehören vor allem das Bleichen, oxidatives Färben und Dauerwellen der Haare, wobei aggressive Oxidations- bzw. Reduktionsmittel, zudem bevorzugt in stark alkalischem Milieu, angewendet werden und dort ihre volle Wirkung zeigen. Aber auch andere chemische Einflüsse entfalten schädigende Wirkungen auf Keratin enthaltendes Material, beispielsweise mit Chlor oder Salzen angereichertes Wasser.

Handelsübliche Spülungen und Kuren enthalten als Aktivsubstanzen hauptsächlich kationische Tenside bzw. Polymere, Wachse und/oder Öle. Je geschädigter das Haar ist, desto mehr anionische Gruppen finden sich an der Oberfläche. Kationische Verbindungen werden auf dieser entgegengesetzt geladenen Oberfläche elektrostatisch angezogen, während Öle und Wachse mit den hydrophoben Gruppen des Keratins wechselwirken. Eine Strukturverbesserung im Haarinneren lässt sich mit diesen Pflegeprodukten daher nicht erreichen.

Der Einsatz von bestimmten ungesättigten Verbindungen, insbesondere Ascorbinsäure, in Haarbehandlungsmitteln für diesen Zweck ist aus der eigenen WO00/57839 bekannt. Ascorbinsäure ist jedoch in wässriger Lösung nicht lange beständig, so dass solche Mittel nicht gelagert werden können, sondern erst kurz vor der Anwendung hergestellt werden müssen.

In der US 2.383.990 wird natives Zein in einem kosmetischen Mittel als Filmbildner bzw. Suspensionsmittel und Oberflächenabdeckungsmaterial (suspending coating material) z. B. für in wäßrig-alkoholischer Lösung unlösliche Pigmente verwendet. Es wird dort ferner als filmbildendes Polymer (Haarlack) in Mitteln zur temporären Formgebung von Haaren wie Stylingmittel und Einlegemittel "Hair setting, waving and/or curling agent" eingesetzt. In US 2.383.990 ist somit die Verwendung von nativem Zein in Mitteln zur vorübergehenden Formgebung von Haaren offenbart, welche keine agressiven haarschädigenden Chemikalien enthalten; nicht offenbart ist hingegen die Verwendung von nativem Zein für die Verbesserung des Haarzustandes, im Sinne der Härtung, Stärkung, Restrukturierung, Reparatur oder Stabilisierung von Haaren oder zur Erhöhung von Glanz, Volumen oder Kämmbarkeit von Haaren.

Die der vorliegenden Erfindung zu Grunde liegende Aufgabe bestand nun darin, ein kosmetisches Haarbehandlungsmittel für die Verwendung zur Verbesserung des Haarzustandes, bereitzustellen, das die vorgenannten Nachteile beseitigt.

Erfindungsgemäß wird diese Aufgabe gelöst durch die Verwendung von nativem Zein in einem Mittel zur zur Härtung, Stärkung, Restrukturierung, Reparatur oder Stabilisierung von strapazierten, geschädigten, empfindlichen, brüchigen und/oder feinen menschlichen Haaren.

Unter nativen Zein wird im Sinne der Erfindung ein nicht-hydrolysiertes Protein verstanden, wie es beispielsweise nach an sich bekannten Extraktions-Methoden aus Mais (*Zea mays*) gewonnen oder kommerziell erhalten werden kann, zum Beispiel von Sigma öder Fluka.

Erst die Entdeckung der Erfinder, dass natives Zein die Härtung, Stärkung, Restrukturierung, Reparatur oder Stabilisierung von Haaren bewirkt was erst durch langwierige Reißkraftmessungen und Kämmkraftmessungen festgestellt wurde - führte zu der erfindungsgemäßen neuen Lehre.

Nur durch die Lehre der vorliegenden Erfindung hatte aber der Fachmann Anlass, natives Zein für die erfindungsgemäße Verwendungen zu benutzen und damit in solchen Haarbehandlungsmitteln zu einzusetzen, in denen Filmbildner üblicherweise nicht enthalten sind oder sogar unüblich und unerwünscht sind, nämlich in Dauerwellmitteln oder in Mittel zur oxidativen Behandlung von Haaren. Erst in Kenntnis der neuen Wirkungen des nativen Zeins wird der Fachmann dieses - im völligen Gegensatz zur bisherigen Vorgehensweise - in solchen Mitteln einsetzen, bei deren Anwendung das Haar besonders stark geschädigt wird und dringend einer Reparatur bedarf. Dies sind nun gerade Haarbehandlungsmittel mit aggressiven Wirkstoffen wie Dauerwellmittel (enthalten haarkeratinreduzierende Mercaptane) oder Mittel zur oxidativen Behandlung von Haaren wie Dauerwellneutralisiermittel, Biondiermittel, Haarbleichmittel und Oxidationshaarfärbemittel (enthalten Persulfat bzw. Wasserstoffperoxid). Dagegen wird das Haar bei Stylingmitteln oder mittels Pigmenten färbenden Stylingmitteln nach US 2.383.990 überhaupt nicht geschädigt.

Überraschenderweise wurde nämlich gefunden, dass durch die Verwendung von nativem Zein die Struktur von keratinischen Fasern (Haaren) derartig verändert wird, dass eine Härtung und Stärkung sowie die Erhöhung der Bruchfestigkeit, Reißfestigkeit oder Bündelzugfestigkeit, insbesondere der strapazierten der geschädigten keratinischen Fasern erfolgt. Neben der noch nachfolgend beschriebenen Pflegewirkung, die aus der Beeinflussung der Haaroberfläche (Cuticula) resultiert, zeigte sich somit insbesondere eine Repair-Wirkung. Diese geht auf Veränderungen im Inneren des Haares (Cortex) zurück. Es wurden die Zugkräfte gemessen, die oxidativ (durch Bleichen) vorgeschädigte Haare zum Zerreißen bringen. Überraschend wurde gefunden, dass diejenigen Haare, die nach der oxidativen Schädigung mit einem natives Zein enthaltenden Mittel behandelt worden waren, eine signifikante Erhöhung der zum Reißen notwendigen Kräfte aufwiesen..

Dies ist überraschend, da aufgrund der Struktur des nativen Zeins weder das Eindringen ins Haarinnere, noch überhaupt eine Beeinflussung der Proteinstrukturen vorhersehbar war. Zudem ist bekannt, dass ungeschädigte nasse Haare deutlich geringere Reißkräfte haben (600 - 900) mN als trockene (1000 - 1500) mN. Somit sollte man erwarten, dass ein Sphäroprotein wie natives Zein die Reißkräfte eher senkt, statt diese, wie gefunden, zu erhöhen.

Damit verbunden wird nicht nur eine Restrukturierung (Repair) von geschädigten keratinischen Fasern ermöglicht, sondern auch ein protektiver Effekt, der einer Schädigung dieser Materialien vor oder während einer Exposition mit entsprechenden Noxen entgegenwirkt und sie zu verhindern oder zu vermindern vermag.

Neben diesen von durch exogene Noxen hervorgerufenen nachteiligen Veränderungen kann die erfindungsgemäße Verwendung auch bei durch physiologische Prozesse bedingte Zustände oder Änderungen der Struktur von keratinischen Fasern vorteilhafte Wirkungen entfalten; beispielsweise bei altersbedingtem brüchigem Haar oder bei feinem Haar, welches angeboren oder altersabhängig erworben sein kann (Babyhaar, Altershaar).

Damit verbunden konnte ferner festgestellt werden, dass bei keratinischen Fasern, insbesondere bei Haaren, durch die erfindungsgemäße Verwendung eine Volumenerhöhung erreicht werden kann, was sich vorteilhaft bei der Herstellung von Frisuren (Erhöhung der Haarfülle) auswirken kann. Es wird vermutet, dass die Wirkung der Volumenerhöhung in kausalem Zusammenhang mit der haarhärtenden, haarstärkenden bzw. haarrestrukturierenden Wirkung der erfindungsgemäß verwendeten Mittel steht.

Gegenstand der vorliegenden Erfindung ist somit die Verwendung gemäß Anspruch 1.

Weitere Ausführungsformen der vorliegenden Erfindung sind in den Ansprüchen wiedergegeben.

Das native Zein, ist zu ca. 40% Bestandteil des Maiskornproteins und zu ca. 60% - 70% im Maiskleber enthalten.

Die Verwendung erfolgt, indem die keratinischen Fasern mit einem das native Zein enthaltenden Mittel in Kontakt gebracht werden und dieses nach Applikation entweder dort verbleibt oder nach einer geeigneten Einwirkungszeit mit einem wässrigen Mittel ab- oder ausgespült wird.

Bevorzugt ist es, wenn das native Zein in einer Menge von 0,001 bis 20,0 Gewichtsprozent, bevorzugt 0,01 bis 10,0 Gewichtsprozent, besonders bevorzugt 0,05 bis 3,0 Gewichtsprozent, jeweils bezogen auf die Gesamtmenge des Mittels, in dem Mittel enthalten ist.

Das für die erfindungsgemäße Verwendung beschriebene Mittel kann in allen geeigneten Formulierungen vorliegen, die in der kosmetischen oder pharmazeutischen Industrie bekannt sind. Insbesondere kann das Mittel als wäßrige oder wäßrig-alkoholische Lösung, als Gel, Creme, Emulsion oder Schaum vorliegen, wobei das Mittel sowohl in Form eines Einkomponentenpräparats als auch in Form eines Mehrkomponentenpräparates konfektioniert sein kann. Im Falle eines Einkomponentenpräparates enthält das Mittel das native Zein zusammen mit geeigneten Hilfs- und Trägerstoffen (beispielsweise Verdickern, Säuren, Duftstoffen, Lösungsmitteln, Salzen, Netzmitteln und/oder UV-Absorbern).

Liegt das Mittel in Form eines Mehrkomponentenpräparates vor, kann dieses aus mindestens zwei verschiedenen, bis zur Anwendung voneinander räumlich getrennten Komponenten bestehen. Eine erste Komponente kann entweder das der vorliegenden Erfindung zugrundeliegende native Zein (Wirkstoff) für sich allein enthalten oder der Wirkstoff kann zusammen mit einem Hilfsstoff (zum Beispiel ein Verdickungsmittel), vorteilhafterweise in fester trockener Form (zum Beispiel als Pulver in verpresster oder nichtverpresster Form, als Granulat oder Tablette), vermischt in dieser ersten Komponente vorliegen. Eine zweite oder weitere Komponente enthält nur Hilfs- und Trägerstoffe.

Es ist aber auch möglich, dass in einem Mehrkomponentenpräparat verschiedene Komponenten verschiedene Wirkstoffe gemäß der vorliegenden Erfindungen einzeln oder als Gemisch enthalten, entweder für sich allein oder zusammen mit verschiedenen Hilfsstoffen vermischt und das die weiteren Komponenten nur Hilfs- und Trägerstoffe enthalten.

Erfindungsgemäß umfasst wird die Verwendung einer Zusammensetzung, die dadurch gekennzeichnet ist, dass sie als Einkomponentenpräparat oder als Mehrkomponentenpräparat vorliegt. Liegt das verwendete Mittel als Mehrkomponentenpräparat vor, so umfasst es eine erste Komponente, die das native Zein mit oder ohne Hilfs- und Zusatzstoffe enthält und eine zweite Komponente, welche die übrigen Bestandteile enthält. Das verwendete Mittel kann ferner als Mehrkomponentenpräparat mit mindestens drei verschiedene Komponenten vorliegen, wobei mindestens eine der Komponenten natives Zein enthält und die übrigen Komponenten die restlichen Bestandteile enthalten.

Es ist selbstverständlich, dass zur Herstellung eines gebrauchsfertigen Mittels die räumlich getrennt vorliegenden einzelnen Komponenten einer Mehrkomponentenpräparation kurz vor deren erfindungsgemäßen Verwendung vermischt werden müssen.

Das erfindungsgemäß verwendete Mittel kann zusätzlich Träger- und Hilfsstoffe, wie zum Beispiel Lösungsmittel wie Wasser, niedere aliphatische Alkohole, beispielsweise Ethanol, n-Propanol und Isopropanol, Glykolether oder Glykole wie Glycerin und insbesondere 1,2-Propandiol; weiterhin Lösungsvermittler, Netzmittel oder Emulgatoren aus den Klassen der anionischen, kationischen, amphoteren oder nichtionogenen oberflächenaktiven Substanzen wie Fettalkoholsulfate, ethoxylierte Fettalkoholsulfate, Alkylsulfonate, Alkylbenzolsulfonate, Alkyltrimethylammoniumsalze, Alkylbetaine, ethoxylierte Fettalkohle, ethoxylierte Nonylphenole, Fettsäurealkanolamide, ferner Verdicker wie höhere Fettalkohole, Stärke oder Cellulosederivate; Salze wie z. B. NaCl; Puffersubstanzen wie Ammoniumhydrogencarbonat; Thiole, Ketocarbonsäuren (Oxocarbonsäuren), insbesondere α-Ketocarbonsäuren, bzw. deren physiologisch verträgliche Salze, UV-Absorber, Parfüme, Farbstoffe, Konditionierer; Haarquellmittel, Konservierungsstoffe, Vaseline, Paraffinöl und Fettsäuren sowie außerdem Pflegestoffe wie kationische Harze, Lanolinderivate, Cholesterin, Pantothensäure und Betain; Treibmittel wie z. B. Propan, Butan, Dimethylether, N₂O und Kohlendioxid, enthalten. Die vorstehend erwähnten Bestandteile werden in den für solche Zwecke üblichen Mengen verwendet, zum Beispiel Wasser in einer Menge von 0,1 bis 95 Gew.%, die Netzmittel und Emulgatoren in Konzentrationen von insgesamt 0,2 bis 30 Gewichtsprozent, die Alkohole in einer Menge von insgesamt 0,1 bis 90 Gewichtsprozent, die Trübungsmittel, Parfümöle, Konservierungsstoffe und Farbstoffe in einer Menge von jeweils 0,01 bis 5 Gewichtsprozent, die Puffersubstanzen in einer Menge von insgesamt 0,1 bis 10 Gewichtsprozent, Lösungsvermittler, Stabilisatoren, sowie haarkonditionierende und haarpflegende Bestandteile in einer Menge von jeweils 0,1 bis 5 Gewichtsprozent, während die Verdickungsmittel und Lösungsvermittler in einer Menge von insgesamt 0,5 bis 20 Gewichtsprozent in diesem Mittel enthalten sein können.

Der pH-Wert des Mittels beträgt bevorzugt 2,0 bis 10,0, insbesondere 3,0 bis 9,0. Erforderlichenfalls kann der gewünschte pH-Wert durch Zugabe von Säuren, beispielsweise α-Hydroxycarbonsäuren wie Milchsäure, Weinsäure, Zitronensäure oder Äpfelsäure, Phosphorsäure, Essigsäure, Glycolsäure Salicylsäure, Glutathion oder Gluconsäurelacton, oder aber alkalisierenden Mitteln wie Ammoniak, Alkanolaminen, Alkylaminen, Alkalihydroxiden, Ammoniumhydroxiden, Alkalicarbonaten, Ammoniumcarbonaten oder Alkaliphosphaten, eingestellt werden.

Bei der Behandlung keratinischer Fasern kann das Mittel dort (beispielsweise im Haar) verbleiben oder nach der Anwendung ausgespült werden. Im letzteren Fall beträgt die Einwirkungszeit des Mittels, je nach Temperatur (etwa 20 bis 60 Grad Celsius, vorzugsweise 30 bis 50 Grad Celsius), 1 bis 60 Minuten, insbesondere 5 bis 20 Minuten, wobei durch Wärmezufuhr die Repairwirkung (Härtung, Restrukturierung und gegebenenfalls die damit verbundene Volumenerhöhung) beschleunigt werden kann; daher ist die Anwendung von Wärme bevorzugt. Nach Beendigung der Einwirkungszeit kann das Haar mit Wasser gespült und gegebenenfalls mit einem Shampoo gewaschen werden.

Bei den für die erfindungsgemäße Verwendung geeigneten Konfektionierungen des Mittels handelt es sich vorzugsweise um oxidative Haarfarben, Haarbleichmittel, oxidative Haartönungen, Haarverformungsmittel wie Dauerwellmittel oder Haarentkräuselungsmittel bzw. Haarstreckungsmittel und Haarglättungsmittel oder Fixierungen. Bevorzugt ist die Verwendung in Haarbehandlungsmitteln, die das Haar durch die Einwirkung ihrer chemischen Bestandteile, insbesondere Reduktionsmittel, Oxidationsmittel oder starke Basen, strapazieren.

Eine vorteilhafte Ausführungsform der Erfindung betrifft daher die Verwendung von Native Zein, vorzugsweise in einer Menge von 0,01 bis 10 Gew.%, besonders bevorzugt in einer Menge von 0,1 bis 5 Gew.%, in einem oxidativen Haarfärbemittel.

Das Haarfärbemittel enthält dann (A) natives Zein und (B) eine oder mehrere Oxidationsfarbstoffvorstufen und/oder eine oder mehrere direktziehende natürliche oder synthetische Farbstoffe. Als Oxidationsfarbstoffvorstufen, bei denen die Färbung unter Einwirkung von Oxidationsmitteln, wie zum Beispiel Wasserstoffperoxid, oder in Gegenwart von Luftsauerstoff erzeugt wird, können beispielsweise die folgenden Entwicklersubstanzen und Kupplersubstanzen und mit sich selbst kuppelnden Verbindungen genannt werden:

### (i) Entwicklersubstanzen:

1,4-Diamino-benzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 4-(2,5-Diaminophenyl)-2-((diethylamino)methyl)thiophen, 2-Chlor-3-(2,5-diaminophenyl)thiophen, 1,4-Diamino-2-(pyridin-3-yl)benzol, 2,5-Diaminobiphenyl, 2,5-Diamino-4'-(1-methylethyl)-1,1'-biphenyl, 2,3',5-Triamino-1,1'-biphenyl, 1,4-Diamino-2-methoxymethyl-benzol, 1,4-Diamino-2-aminomethyl-benzol, 1,4-Diamino-2-((phenylamino)methyl)benzol, 1,4-Diamino-2-((ethyl-(2-hydroxyethyl)-amino)methyl)benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylaminoanilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)aminol-anilin, 4-[Di(2-hydroxyethyl)amino]-2-methyl-anilin, 4-[(2-Methoxyethyl)amino]-anilin, 4-[(3-Hydroxypropyf)amino]-anilin, 4-[(2,3-Dihydroxypropyl)amino]-anilin, 4-(((4-Aminophenyl)-methyl)amino)anilin, 4-[(4-Amino-phenylamino)-methyl]-phenol, 1,4-Diamino-N-(4-pyrrolidin-1-yl-benzyl)-benzol, 1,4-Diamino-N-furan-3-ylmethyl-benzof, 1,4-Diamino-N-thiophen-2-ylmethyl-benzol, 1,4-Diamino-N-furan-2-ylmethyl-benzol, 1,4-Diamino-N-thiophen-3-ylmethyl-benzol, 1,4-Diamino-N-benzyl-benzol, 1,4-Diamino-2-(1-hydroxyethyl)-benzol, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 2,5-Diamino-4'-hydroxy-1,1'-biphenyl, 2,5-Diamino-2'-trifluormethyl-1,1'-biphenyl, 2,4',5-Triamino-1,1'-biphenyl, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1H-pyrazol, 4,5-Diamino-1-pentyl-1H-pyrazol, 4,5-Diamino-1-(phenylmethyl)-1H-pyrazol, 4,5-Diamino-1-((4-methoxyphenyl)methyl-1H-pyrazol, 2-Amino-phenol, 2-Amino-6-methyl-phenol, 2-Amino-5-methyl-phenol, 1,2,4-Trihydroxy-benzol, 2,4-Diaminophenol, 1,4-Dihydroxybenzol, 2-(((4-Aminophenyl)amino)-methyl)-1,4-diaminobenzol, alleine oder im Gemisch miteinander.

### (ii) Kupplersubstanzen:

N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methylphenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxyindolin, 5-Hydroxy-indol, 6-Hydroxy-indol, 7-Hydroxy-indol, 2,3-Indolindion, alleine oder im Gemisch miteinander.

### (iii) Mit sich selbst kuppelnde Verbindungen:

2-Amino-5-methylphenol, 2-Amino-6-methylphenol, 2-Amino-5-ethoxyphenol oder 2-Propyl-amino-5-aminopyridin.

Die Gesamtmenge der im Haarfärbemittel enthaltenen Oxidationsfarbstoffvorstufen beträgt etwa 0,01 bis 12 Gewichtsprozent, insbesondere etwa 0,2 bis 6 Gewichtsprozent.

Zur Erzielung bestimmter Farbnuancen können ferner auch übliche natürliche oder synthetische (nicht-oxidative) direktziehende Farbstoffe, beispielsweise Triphenylmethanfarbstoffe, aromatische Nitrofarbstoffe, Azofarbstofte, Chinonfarbstoffe, kationische oder anionische Farbstoffe, Henna oder Indigo in dem Haarfärbemittel enthalten sein.

Darüberhinaus können in dem Haarfärbemittel Antioxidantien wie zum Beispiel Ascorbinsäure, Thioglykolsäure oder Natriumsulfit, sowie Komplexbildner für Schwermetalle, beispielsweise Ethylendiaminotetraacetat oder Nitriloessigsäure, in einer Menge von bis zu etwa 0,5 Gewichtsprozent enthalten sein. Parfümöle können in der Farbträgermasse in einer Menge von bis zu etwa 1 Gewichtsprozent enthalten sein.

Selbstverständlich kann das Haarfärbemittel gegebenenfalls weitere, für Haarfärbemittel übliche Zusätze, wie zum Beispiel Konservierungsstoffe und Parfümöle; Antioxidantien, beispielsweise Natriumsulfit, Thioglykolsäure oder Ascorbinsäure; Komplexbildner; Lösungsmittel wie Wasser, niedere aliphatische Alkohole, beispielsweise Ethanol, Propanol und Isopropanol, oder Glykole wie Glycerin und 1,2-Propylenglykol; Netzmittel oder Emulgatoren aus den Klassen der anionischen, kationischen, amphoteren oder nichtionogenen oberflächenaktiven Substanzen; Verdicker wie höhere Fettalkohole, Stärke oder Cellulosederivate; weiterhin Weichmacher; Vaseline; Silikonöle, Paraffinöl und Fettsäuren sowie kationische Harze, wie zum Beispiel Kämmbarkeitsverbesserer und gegen Haarflattem (hair fizz) wirkende Stoffe wie kationische Polymere, z. B. CTFA: POLYQUATERNIUM-1, CTFA: POLYQUATERNIUM-4, CTFA: POLYQUATERNIUM-5, CTFA: POLYQUATERNIUM-6, CTFA: POLYQUATERNIUM-7, CTFA: POLYQUATERNIUM-10, CTFA: POLYQUATERNIUM-11, CTFA: POLYQUATERNIUM-16, CTFA: POLYQUATERNIUM-22, CTFA: POLYQUATERNIUM-32, CTFA: POLYQUATERNIUM-35, CTFA: POLYQUATERNIUM-36, CTFA: POLYQUATERNIUM-37, CTFA: POLYQUATERNIUM-39, CTFA: POLYQUATERNIUM-44, CTFA: POLYQUATERNIUM-45, CTFA: POLYQUATERNIUM-46, CTFA: POLYQUATERNIUM-47, CTFA: POLYSILICONE-3, CTFA: POLYSILICONE-4, CTFA: POLYSILICONE-5, CTFA: POLYSILICONE-6, CTFA: POLYSILICONE-7 CTFA: POLYSILICONE-8 und CTFA: POLYSILICONE-1; Lanolinderivate, Cholesterin, Vitamine, Pantothensäure und Betain, enthalten. Die Abkürzung "CTFA" steht für *International Cosmetic Ingredient Dictionary and Handbook,* Eighth Edition 2000 (ISBN 1-882621-22-0)

Die erwähnten Bestandteile werden in den für solche Zwecke üblichen Mengen verwendet, zum Beispiel die Netzmittel und Emulgatoren in Konzentrationen von 0,1 bis 30 Gewichtsprozent, die Verdicker in einer Menge von 0,1 bis 25 Gewichtsprozent und die Pflegestoffe in einer Konzentration von 0,1 bis 5,0 Gewichtsprozent.

Besonders vorteilhaft ist hierbei der Zusatz von nicht-ionischen und/oder anionischen Tensiden oder Emulgatoren, wie zum Beispiel Fettalkoholsulfaten, insbesondere Laurylsulfat und Natriumcocoylsulfat, oxethylierten Fettalkoholsulfaten, insbesondere Natriumlaurylethersulfaten mit 2 bis 4 Ethylenoxideinheiten im Molekül, oxethylierten Fettsäureestern, oxethylierten Nonylphenolen, oxethylierten Fettalkoholen, Alkylbenzolsulfonaten oder Fettsäurealkanolamiden, in einer Gesamtmenge von etwa 0,1 bis 30 Gewichtsprozent, vorzugsweise 0,2 bis 15 Gewichtsprozent.

Der pH-Wert des Haarfärbemittels auf der Basis von Oxidationsfarbstoffvorstufen liegt in einem Bereich von etwa 6 bis 12, vorzugsweise 9 bis 11, wobei der pH-Wert des gebrauchsfertigen Oxidationshaarfärbemittels (das heißt der Mischung des erfindungsgemäßen Haarfärbemittels mit dem Oxidationsmittel) etwa 5,5 bis 10, vorzugsweise 6 bis 9, beträgt. Je nach Zusammensetzung und gewünschtem pH-Wert des Haarfärbemittels erfolgt die Einstellung des pH-Wertes vorzugsweise mit Ammoniak oder organischen Aminen, wie zum Beispiel Glucaminen, Aminomethyl-propanol, Monoethanolamin oder Triethanolamin, anorganischen Basen, beispielsweise Natriumhydroxid, Kaliumhydroxid, Natriumcarbonat oder Calciumhydroxid, beziehungsweise organischen oder anorganischen Säuren, wie zum Beispiel Milchsäure, Zitronensäure, Essigsäure oder Phosphorsäure.

Das oxidative Haarfärbemittel bzw. oxidative Haartönungsmittel wird vorzugsweise in Form einer wässrigen oder wässrig-alkoholischen Zubereitung, beispielsweise als verdickte Lösung, als Emulsion, als Creme oder als Gel, konfektioniert. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Für die Anwendung zur oxidativen Färbung vermischt man das vorstehend beschriebene Haarfärbemittel bzw. Haartönungsmittel unmittelbar vor dem Gebrauch mit einem Oxidationsmittel und trägt eine für die Färbung ausreichende Menge, in der Regel etwa 60 bis 200 Gramm, der gebrauchsfertigen Zubereitung auf das Haar auf.

Als Oxidationsmittel zur Entwicklung der Färbung kommen hauptsächlich Wasserstoffperoxid oder dessen Additionsverbindungen an Harnstoff, Melamin oder Natriumborat in Form einer 1- bis 12prozentigen, vorzugsweise 1,5- bis 6prozentigen wässrigen Lösung in Betracht. Das Mischungsverhältnis von Haarfärbemittel zu Oxidationsmittel ist abhängig von der Konzentration des Oxidationsmittels und beträgt in der Regel etwa 5:1 bis 1:2, vorzugsweise 1:1, wobei der Gehalt an Oxidationsmittel in der gebrauchsfertigen Zubereitung vorzugsweise etwa 0,5 bis 8 Gewichtsprozent, insbesondere 1 bis 4 Gewichtsprozent, beträgt.

Man lässt das gebrauchsfertige Haarfärbemittel bei 15 bis 50 °C etwa 10 bis 45 Minuten, vorzugsweise etwa 15 bis 30 Minuten lang, auf die Haare einwirken, spült sodann die Haare mit Wasser aus und trocknet sie. Gegebenenfalls wird im Anschluss an diese Spülung mit einem Shampoo gewaschen und eventuell mit einer schwachen organischen Säure, wie zum Beispiel Weinsäure, nachgespült. Anschließend wird das Haar getrocknet.

Das native Zein kann auch in einem Vorbehandlungsmittel vor chemischen und/oder physikalischen Behandlungen von keratinischen Fasern, insbesondere einer oxidativen Haarfärbung, einer oxidativen Haartönung, einer Haarbleichung oder vor einer Haardauerverformung, verwendet werden, um einer Haarschädigung durch diese oxidativen Behandlungen vorzubeugen.

Eine vorteilhafte Ausführungsform der Erfindung betrifft daher die Verwendung von Nativem Zein, vorzugsweise in einer Menge von 0,01 bis 10 Gew.%, besonders bevorzugt in einer Menge von 0,1 bis 5 Gew.%, in einem Vorbehandlungsmittel, vor der Anwendung eines kosmetischen Mittels, das agressive Chemikalien wie Reduktionsmittel oder Oxidationsmittel enthält, vorzugsweise in einem Dauerwellvorbehandlungsmittel. Das Dauerwellvorbehandlungsmittel wird vor der Anwendung der Dauerwellmittel (Reduktionsmittel und oxidative Fixierung) mit den Haaren, besonders den Haarspitzen, in Kontakt gebracht. Das native Zein versiegelt bevorzugt die besonders geschädigten Stellen des Haares - insbesondere die Haarspitzen - und schützt sie so vor der Einwirkung des danach anzuwendenden reduzierenden Dauerwellmittels.

In einer weiteren Ausführungsform wird das native Zein in einem Haardauerverformungsmittel (Dauerwellmittel oder Haarentkräuselungsmittel) oder in einem Mittel zur oxidativen Behandlung von Haaren (z. zum Bleichen, Hellerfärben oder Fixieren von Haaren) verwendet.

Das Haardauerverformungsmittel enthält dann (A) native Zein, vorzugsweise in einer Menge von 0,01 bis 10 Gew.%, besonders bevorzugt in einer Menge von 0,1 bis 5 Gew.%, und (B) mindestens einen haarkeratinreduzierenden Stoff.

Die im Haarverformungsmittel verwendbaren Verformungswirkstoffe sind solche auf der Basis üblicher haarkeratinreduzierender Stoffe, wie zum Beispiel Salze der schwefeligen Säure oder bestimmte Mercaptoverbindungen, insbesondere Salze oder Ester von Mercaptocarbonsäuren. Das Haardauerverformungsmittel enthält die keratinreduzierenden Verbindungen in den für die Haarverformung üblichen Mengen, beispielsweise die Ammoniumsalze der Thioglykolsäure oder Thiomilchsäure oder aber Cystein, in einer Konzentration von 6 bis 12 Gewichtsprozent. Der pH-Wert der alkalischen Verformungsmittel liegt im allgemeinen bei 7 bis 10, wobei die Einstellung vorzugsweise mit Ammoniak, Monoethanolamin, Ammoniumcarbonat oder Ammoniumhydrogencarbonat erfolgt. Ist die Verformungskomponente sauer (zum Beispiel auf pH = 6,5 bis 6,9) eingestellt, werden Ester von Mercaptocarbonsäuren, wie beispielsweise Monothioglykolsäureglykolester oder Monothioglykolsäureglycerinester, vorzugsweise aber Mercaptoacetamide oder 2-Mercaptopropionsäureamide, in einer Konzentration von 2 bis 14 Gewichtsprozent; oder aber die Salze der schwefeligen Säure, zum Beispiel Natrium-, Ammonium- oder Monoethanolammoniumsulfit, in einer Konzentration von 3 bis 8 Gewichtsprozent (berechnet als SO₂), verwendet. Bevorzugt ist die verwendete haarkeratinreduzierende Verbindung das Salz oder das Derivat einer Mercaptocarbonsäure. Besonders bevorzugt ist die keratinreduzierende Verbindung ausgewählt aus Thioglykolsäure, Cystein und Thiomilchsäure oder deren Salzen.

Zur Wirkungssteigerung können dem Haardauerverformungsmittel Quell- und Penetrationsstoffe, wie beispielsweise Harnstoff, mehrwertige Alkohole, Ether, Melamin, Alkali- oder Ammoniumthiocyanat, Isopropanol, Imidazolidin-2-on, 2-Pyrrolidon und 1-Methyl-2-pyrrolidon, in einer Konzentration von etwa 0,5 bis 50 Gewichtsprozent, vorzugsweise 2 bis 30 Gewichtsprozent, zugesetzt werden.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur 3 bis 30 Minuten, bevorzugt 2 bis 20 Minuten, beträgt, wird das Haar, vorzugsweise ohne dass das Verformungsmittel zuvor mit Wasser ausgespült wird, oxidativ fixiert.

Das Mittel zur oxidativen Behandlung von Haaren ist gekennzeichnet durch den Gehalt an (A) nativem Zein und (B) mindestens einem Oxidationsmittel..

Es wird, je nach Haarfülle, in einer Menge von etwa 50 bis 200 g verwendet. Für die Fixierung kann jedes beliebige, bisher in Fixiermitteln verwendete Oxidationsmittel verwendet werden. Beispiele für solche Oxidationsmittel sind Kaliumbromat, Natriumbromat, Natriumperborat, Dehydroascorbinsäure, Hydrogenperoxid und Harnstoffperoxid. Die Konzentration der Oxidationsmittel ist in Abhängigkeit von Anwendungszeit (in der Regel 1 bis 40 Minuten, bevorzugt 5 bis 20 Minuten) und Anwendungstemperatur (25 bis 50 Grad Celsius) unterschiedlich. Normalerweise werden in den wässrigen Fixiermitteln die Oxidationsmittel in einer Konzentration von etwa 0,5 bis 12,0 Gewichtsprozent verwendet. Das Fixiermittel kann selbstverständlich weitere Stoffe, wie zum Beispiel schwache Säuren oder Peroxidstabilisatoren, enthalten.

Sowohl das Haardauerverformungsmittel als auch das Mittel zur oxidativen Behandlung von Haaren (z. B. das Fixiermittel) können in Form einer wässrigen Lösung oder einer Emulsion sowie in verdickter Form auf wässriger Basis, insbesondere als Creme, Gel oder Paste, vorliegen.

Besonders bevorzugt liegt die Fixierung in viskoser Form mit einer Konsistenz vor, welche es ermöglicht, das Haar der Kundin im Sitzen und nicht wie gewöhnlich im Waschbecken zu fixieren. Bevorzugt ist das Fixiermittel eine oxidationsmittelhaltige viskose Zubereitung mit einer Viskosität von 100 bis 10.000 mPa • s bei 25 Grad Celsius, wobei eine Viskosität von 300 bis 1000 mPa • s bei 25 Grad Celsius besonders bevorzugt ist. Die Viskositätsangaben beziehen sich auf die Messung mit einem Haake RotationsViskosimeter Typ VT 501 bei einer Schergeschwindigkeit von 64,5 pro Sekunde.

Ebenfalls ist es möglich, diese Mittel unter Druck in Aerosoldosen abzufüllen und daraus als Aerosolschaum zu entnehmen.

Selbstverständlich können sowohl das Haardauerverformungsmittel als auch das Mittel zur oxidativen Behandlung von Haaren alle für derartige Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure, Cellulosederivate, Alginate, Vaseline oder Paraffinöl, Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quartäre Ammoniumsalze, Alkylbetaine, oxethylierte Fettalkohole, oxethylierte Alkylphenole, Fettsäurealkanolamide oder oxethylierte Fettsäureester, ferner Trübungsmittel, wie zum Beispiel Polyethylenglykolester, oder Alkohole, wie beispielsweise Ethanol, Propanol, Isopropanol oder Glycerin, Lösungsvermittler, Stabilisatoren, Puffersubstanzen, Parfümöle, Haarkonditionierungsmittel wie zum Beispiel Kämmbarkeitsverbesserer und gegen Haarflattern (hair fizz) wirkende Stoffe wie kationische Polymere, z. B. CTFA: POLYQUATERNIUM-1, CTFA: POLYQUATERNIUM-4, CTFA: POLYQUATERNIUM-5, CTFA: POLYQUATERNIUM-6, CTFA: POLYQUATERNIUM-7, CTFA: POLYQUATERNIUM-10, CTFA: POLYOUATERNIUM-11, CTFA: POLYQUATERNIUM-16, CTFA: POLYQUATERNIUM-22, CTFA: POLYQUATERNIUM-32, CTFA: POLYQUATERNIUM-35, CTFA: POLYQUATERNIUM-36, CTFA: POLYQUATERNIUM-37, CTFA: POLYQUATERNIUM-39, CTFA: POLYQUATERNIUM-44, CTFA: POLYQUATERNIUM-45, CTFA: POLYQUATERNIUM-46, CTFA: POLYOUATERNIUM-47, CTFA: POLYSILICONE-3, CTFA: POLYSILICONE-4, CTFA: POLYSILICONE-5, CTFA: POLYSILICONE-6, CTFA: POLYSILICONE-7 CTFA: POLYSILICONE-8 und CTFA: POLYSILICONE-13;sowie haarpflegende Bestandteile, wie zum Beispiel Lanolinderivate, Cholesterin, Pantothensäure, Creatin oder Betain, enthalten. Ferner können diesen Mitteln optische Aufheller in Form von Cumarin-, Stilben-, Naphthalimid-, Benzoxazol- oder Styrylderivaten zugesetzt werden.

Die erwähnten Bestandteile werden in den für solche Zwecke üblichen Mengen verwendet, zum Beispiel die Netzmittel und Emulgatoren in Konzentrationen von etwa 0,2 bis 30 Gewichtsprozent, während die Verdickungsmittel in einer Menge von etwa 0,1 bis 25 Gewichtsprozent, bezogen jeweils auf die gebrauchsfertigen Mittel, enthalten sein können.

Anschließend werden die Wickler entfernt, das Fixiermittel mit Wasser aus dem Haar ausgespült und das Haar in üblicher Weise weiterbehandelt. Vorteilhaft wird das Haar im Anschluss an die Dauerverformung zur Wasserwelle gelegt.

Es konnte festgestellt werden, dass die erfindungsgemäße Verwendung eines Mittels mit einem Gehalt an nativem Zein gemäß der vorliegenden Erfindung eine deutliche Strukturverbesserung zuvor geschädigter keratinischer Fasern ermöglicht, die sich zudem mit einer statistisch hochsignifikanten Erhöhung der Reißkraft nachweisen lässt.

### Messung der Repairwirkung

Die Ermittlung der Reißkraft von Haaren, die ein Indikator für die strukturelle Integrität des Haarcortex und damit ein Maß für den Schädigungsgrad ist, erfolgt durch für diese Zwecke übliche Zug-Dehnungs-Messungen. Von jeder Haarsträhne werden 20 Einzelhaare ausgewählt und die einzelnen Haardurchmesser mit einem computergesteuerten Lasermikrometer bestimmt. Anschließend wird mit einem Zug-Dehnungs-Meßgerät (MTT 160/600 Series Miniature Tensile Tester, Serial No. 600.95.05.001, Fa. DIA-STRON Ltd., England) die Kraft gemessen, die nötig ist, um die einzelnen Haare zum Zerreißen zu bringen. Aus diesen einzelnen, aufgrund der unterschiedlichen Haardurchmesser voneinander abweichenden Reißkraft-Meßwerten wird die sog. Bündelzugfestigkeit (BZF) ermittelt, indem aus den Einzelwerten unter Berücksichtigung der jeweiligen Haardurchmesser die Reißkraft für einen Haardurchmesser von 0,08 mm (mittlerer Durchmesser) errechnet wird. Durch Einbeziehung der Haardichte erfolgt schließlich die Umrechnung in die Einheit der Bündelzugfestigkeit (cN/tex). Je größer der Zahlenwert der Bündelzugfestigkeit, desto geringer ist die Haarschädigung.

Die Messungen an Haaren, die mit natives zeinhaltigen und natives zeinfreiem Dauerwellvorbehandlungsmittel in Form eines Shampoos behandelt wurden, ergaben folgende Ergebnisse:

Geschädigtes Haar (blondiert) wurde behandelt mit dem Shampoo nach Beispiel 1 jedoch ohne Zein (die Menge an Native Zein wurde durch Wasser ersetzt): BZF = 10,51 +/- 0,4 cN/tex (ermittelt an 18 Haaren aus einer Shampoo-behandelten Strähne);

Geschädigtes Haar (blondiert) behandelt mit dem Shampoo nach Beispiel 1 mit 2 Gew.% Native Zein: BZF = 12,36 +/- 0,4 cN/tex (ermittelt an 24 Haaren aus einer Shampoo-behandelten Strähne).

Der Unterschied zwischen den oben angegebenen Mittelwerten ist statistisch hochsignifikant (Signifikanzniveau ermittelt mit t-Test: 99,9%). Die Bündelzugfestigkeit wird durch den Gehalt an 2 Gew.% Native Zein von 10,51 cN/tex auf 12,36 cN/tex erhöht; dies entspricht einem Anstieg um 17,6 %. Der Zusatz von Native Zein bedingt somit eine deutliche Haarstärkung bzw. Repairwirkung.

### Messung der Kämmbarkeitsverbesserung

Die Kämmbarkeit des Haares ist ebenfalls ein wichtiger Parameter zur Beschreibung der Haarqualität. Verschiedene äußere Einwirkungen wie bestimmte kosmetische Behandlungen (Bleichen, Färben, Dauerwellen), Bewetterung, häufiges Kämmen und Bürsten verschlechtern die Kämmbarkeit des Haares, was auf eine Schädigung der Cuticula zurückzuführen ist.

Das Prinzip der meisten Verfahren zur messtechnischen Erfassung der Kämmbarkeit besteht darin, die Kraft (Kämmkraft) zu messen, die nötig ist, um einen Kamm unter exakt definierten Randbedingungen durch eine Haarsträhne zu kämmen.

Im Rahmen der eigenen Untersuchung wurde dazu eine automatisierte Apparatur verwendet, bei der ein mechanischer Greifarm die zu untersuchenden Strähnen aus einem Lager holt und auf den Haken einer Kraftmessdose hängt. Dann werden die Strähnen nacheinander mehrmals automatisch mit konstanter Geschwindigkeit gekämmt und für jeden Kämmvorgang wird die Kämmkraft N (Newton) als Funktion der Kämmstrecke (Strähnenlänge) aufgenommen. Die angegebenen Kämmkraftwerte ergeben sich schließlich aus einer Mittelwertbildung der Kämmkräfte über die Kämmstrecke. Je geringer die Kämmkraft ist, desto besser ist die Kämmbarkeit des Haares.

Die Messungen an Haaren, die mit zeinhaltigen und zeinfreien Shampoos behandelt wurden, ergaben folgende Ergebnisse:

Geschädigtes Haar (blondiert) wurde behandelt mit dem Shampoo nach Beispiel 1 jedoch ohne Zein (die Menge an Native Zein wurde durch Wasser ersetzt) Die Kämmkraft betrug 1,43 +/- 0,05 N (gemessener Mittelwert aus 3 mit Shampoo behandelten Haarsträhnen).

Geschädigtes Haar (blondiert) behandelt mit Shampoo nach Beispiel 1 mit 2 Gew.% Native Zein: Die Kämmkraft betrug 1,21 +/- 0,08 N (gemessener Mittelwert aus 3 mit Shampoo behandelten Haarsträhnen).

Der Unterschied zwischen den oben angegebenen Mittelwerten ist statistisch signifikant (Signifikanzniveau ermittelt mit t-Test: 97,5%).

Die Kämmkraft verringert sich durch den Gehalt an 2 Gew.% Native Zein von 1,43 N auf 1,21 N, also um 15,3 %. Der Zusatz von Native Zein bedingt somit eine deutlich feststellbare Verbesserung der Nasskämmbarkeit des Haares.

Alle in der vorliegenden Beschreibung genannten Prozentangaben stellen, soweit nicht anders angegeben, Gewichtsprozente, bezogen auf das Gesamtgewicht der jeweiligen Zusammensetzung, dar.

Die nachfolgenden Beispiele sollen den Gegenstand näher erläutern.

### BEISPIELE

### Beispiel 1: Dauerwellvorbehandlungsmittel (Shampoo)

| | |
|---|---|
| Natriumlaurylethersulfat (25%ige wässrige Lösung) | 40,0 Gew.% |
| Cocamidopropyl Betain | 5,0 Gew.% |
| NaCl | 2,0 Gew.% |
| natives Zein | 2,0 Gew.% |
| Wasser | ad 100,0 Gew.% |

### Beispiel 2: Haarshampoo zur Dauerwellvorbehandlung

| | |
|---|---|
| Natriumlaurylethersulfat (25%ige wässrige Lösung) | 35,0 Gew.% |
| NaCl | 3,0 Gew.% |
| Triethanolamin | 4,0 Gew.% |
| 1,2-Dibrom-2,4-dicyanobutamin-2-phenoxyethanol | 0,1 Gew.% |
| Parfümöl | 0,1 Gew.% |
| native Zein | 1,0 Gew.% |
| Wasser | ad 100,0 Gew.% |

### Beispiel 3: Oxidationshaarfärbemittel in Cremeform

| | |
|---|---|
| Stearylalkohol | 8,000 Gew.% |
| Paraffinöl | 13,000 Gew.% |
| Wollfett | 6,000 Gew.% |
| Parfüm | 0,300 Gew.% |
| 4-Aminophenol | 1,360 Gew.% |
| 1-Naphthol | 0,500 Gew.% |
| Resorcin | 0,014 Gew.% |
| 2-Amino-6-chlor-4-nitrophenol | 0,003 Gew.% |
| Ammoniak, 25 %ige wässrige Lösung | 12,000 Gew.% |
| Ethylendiaminoteraacetat-Dinatriumsalz | 1,000 Gew.% |
| Ascorbinsäure | 1,000 Gew.% |
| natives Zein | 1,000 Gew.% |
| Wasser | ad 100,000 Gew.% |

50 g des vorstehenden Haarfärbemittels werden unmittelbar vor Gebrauch mit 50 g einer 12prozentigen wässrigen Wasserstoffperoxidlösung vermischt. Das Gemisch wird sodann auf blonde Naturhaare aufgetragen und nach einer Einwirkungszeit von 30 Minuten bei 40 °C mit Wasser wieder aus dem Haar ausgespült. Nach dem Trocknen der Haare wird eine leuchtende, kupferrote Färbung erhalten.

### Beispiel 4: Dauerwellmittel

| | |
|---|---|
| Thioglykolsäure (80%ige wässrige Lösung) | 9,5 Gew.% |
| Ammoniak (25% ige wässrige Lösung) | 1,6 Gew.% |
| Ammoniumcarbonat | 4,5 Gew.% |
| natives Zein | 2,0 Gew.% |
| Parfümöl | 0,2 Gew.% |
| Wasser | ad 100,0 Gew.% |

### Beispiel 5: Dauerwellfixierung

| | |
|---|---|
| Wasserstoffperoxid | 4,6 Gew.% |
| Zitronensäure | 0,2 Gew.% |
| natives Zein | 2,0 Gew.% |
| Parfümöl | 0,1 Gew.% |
| Wasser | ad 100,0 Gew.% |

## Patentansprüche

1. Verwendung von nativem Zein (d. h. als nicht-hydrolysiertes Protein) in einem Mittel als Wirkstoff zur Härtung, Stärkung, Restrukturierung, Reparatur oder Stabilisierung von strapazierten, geschädigten, empfindlichen, brüchigen und/oder feinen menschlichen Haaren.

2. Verwendung nach Anspruch 1, **dadurch gekennzeichnet, dass** das Mittel vor, während oder nach einer Exposition des Haares mit chemischen und/oder physikalischen Noxen mit den Haaren in Kontakt gebracht wird.

3. Verwendung nach Anspruch 2 in einem Vorbehandlungsmittel vor einer chemischen und/oder physikalischen Behandlung von Haaren.

4. Verwendung nach einem der Ansprüche 1 bis 3 zur Verhütung oder Verminderung von Schädigungen der inneren Struktur oder zur Reparatur (Restrukturierung) von Haaren.

5. Verwendung nach Anspruch 3, **dadurch gekennzeichnet, dass** die chemische Behandlung eine oxidative Färbung, Bleichung oder Dauerverformung umfasst.

6. Verwendung nach einem der Ansprüche 1 bis 5, **dadurch gekennzeichnet, dass** das Zein in einer Menge von 0,001 bis 20,0 Gewichtsprozent, bezogen auf die Gesamtmenge, in dem Mittel enthalten ist.

7. Verwendung nach einem der Ansprüche 1 bis 6, **dadurch gekennzeichnet, dass** das Zein in einer Menge von 0,05 bis 3,0 Gewichtsprozent, bezogen auf die Gesamtmenge, in dem Mittel enthalten ist.

8. Verwendung nach einem der Ansprüche 1 bis 7, **dadurch gekennzeichnet, dass** das kosmetische Mittel als Lösung, Emulsion, Schaum, Creme oder Gel vorliegt.

9. Verwendung nach einem der Ansprüche 1 bis 8, **dadurch gekennzeichnet, dass** das Mittel für eine Dauer von 1 bis 60 Minuten bei einer Temperatur zwischen 20°C und 60°C mit den Haaren in Kontakt gebracht wird.

10. Verwendung von nativem Zein (d. h. als nicht-hydrolysiertes Protein) zur Härtung, Stärkung, Restrukturierung, Reparatur oder Stabilisierung des Haares in einem Haarbehandlungsmittel, welches das Haar durch die Einwirkung seiner chemischen Bestandteile strapaziert.

11. Verwendung nach Anspruch 10 in einem Dauerwellmittel mit einem Gehalt an
(A) nativem Zein und
(B) mindestens einem haarkeratinreduzierenden Stoff.

12. Verwendung nach Anspruch 10 in einem Mittel zur oxidativen Behandlung von Haaren mit einem Gehalt an
(A) nativem Zein und
(B) mindestens einem Oxidationsmittel.

13. Verwendung nach Anspruch 12, **dadurch gekennzeichnet, dass** das Oxidationsmittel ausgewählt ist aus Kaliumbromat, Natriumbromat, Natriumperborat, Dehydroascorbinsäure, Hydrogenperoxid und Harnstoffperoxid.

14. Verwendung nach Anspruch 10 in einem Haarfärbemittel, enthaltend
(A) natives Zein und
(B) eine oder mehrere Oxidationsfarbstoffvorstufen.

## Claims

1. Use of native zein (i.e. as non-hydrolysed protein) in a composition as an active substance for hardening, reinforcing, restructuring, repairing or stabilizing stressed, damaged, sensitive, fragile and/or fine human hair.

2. Use according to Claim 1, **characterized in that** the composition is brought into contact with the hair before, during or after exposure of the hair to chemical and/or physical noxae.

3. Use according to Claim 2 in a pretreatment composition before a chemical and/or physical treatment of hair.

4. Use according to one of Claims 1 to 3 for preventing or reducing damage to the inner structure of or for repairing (restructuring) hair.

5. Use according to Claim 3, **characterized in that** the chemical treatment comprises an oxidative colouring, bleaching or permanent shaping.

6. Use according to one of Claims 1 to 5, **characterized in that** the composition comprises the zein in an amount of from 0.001 to 20:0 per cent by weight, based on the total amount.

7. Use according to one of Claims 1 to 6, **characterized in that** the composition comprises the zein in an amount of from 0.05 to 3.0 per cent by weight, based on the total amount.

8. Use according to one of Claims 1 to 7, **characterized in that** the cosmetic composition is in the form of a solution, emulsion, foam, cream or gel.

9. Use according to one of Claims 1 to 8, **characterized in that** the composition is brought into contact with the hair for a duration of from 1 to 60 minutes at a temperature of between 20 °C and 60 °C.

10. Use of native zein (i.e. as non-hydrolysed protein) for hardening, reinforcing, restructuring, repairing or stabilizing hair, in a hair treatment composition which stresses the hair due to the action of its chemical constituents.

11. Use according to Claim 10 in a permanent wave composition having a content of
(A) native zein and
(B) at least one hair keratin-reducing substance.

12. Use according to Claim 10 in a composition for oxidative treatment of hair having a content of
(A) native zein and
(B) at least one oxidizing agent.

13. Use according to Claim 12, **characterized in that** the oxidizing agent is chosen from potassium bromate, sodium bromate, sodium perborate, dehydroascorbic acid, hydrogen peroxide and urea peroxide.

14. Use according to Claim 10 in a hair colouring composition comprising
(A) native zein and
(B) one or more oxidizing dyestuff precursors.

## Revendications

1. Utilisation de zéine native (c'est-à-dire sous forme de protéine non hydrolysée) dans une composition, en tant que substance active destinée au durcissement, au renforcement, à la restructuration, à la réparation ou à la stabilisation de cheveux humains fatigués, abîmés, sensibles, cassants et/ou fins.

2. Utilisation selon la revendication 1, **caractérisée en ce que** la composition est mise en contact avec les cheveux avant, pendant ou après une exposition du cheveu à des agents chimiques et/ou physiques nocifs.

3. Utilisation selon la revendication 2, dans une composition de prétraitement avant un traitement chimique et/ou physique des cheveux.

4. Utilisation selon l'une quelconque des revendications 1 à 3, pour la prévention ou la réduction d'endommagements de la structure interne ou pour la réparation (restructuration) des cheveux.

5. Utilisation selon la revendication 3, **caractérisée en ce que** le traitement chimique comprend une teinture par oxydation, une décoloration ou une mise en forme permanente.

6. Utilisation selon l'une quelconque des revendications 1 à 5, **caractérisée en ce que** la zéine est contenue dans la composition en une quantité de 0,001 à 20,0 % en poids, par rapport à la quantité totale.

7. Utilisation selon l'une quelconque des revendications 1 à 6, **caractérisée en ce que** la zéine est contenue dans la composition en une quantité de 0,05 à 3,0 % en poids, par rapport à la quantité totale.

8. Utilisation selon l'une quelconque des revendications 1, à 7, **caractérisée en ce que** la composition cosmétique se trouve sous forme de solution, émulsion, mousse, crème ou gel.

9. Utilisation selon l'une quelconque des revendications 1 à 8, **caractérisée en ce que** la composition est mise en contact avec les cheveux pendant une durée de 1 à 60 minutes, à une température comprise entre 20 °C et 60 °C.

10. Utilisation de zéine native (c'est-à-dire sous forme de protéine non hydrolysée) pour le durcissement, le renforcement, la restructuration, la réparation ou la stabilisation du cheveu dans une composition de traitement capillaire qui fatigue le cheveu sous l'effet de ses composants chimiques.

11. Utilisation selon la revendication 10, dans une composition d'ondulation permanente ayant une teneur en
(A) zéine native et
(B) au moins une substance réduisant la kératine du cheveu.

12. Utilisation selon la revendication 10, dans une composition destinée au traitement oxydant des cheveux, ayant une teneur en
(A) zéine native et
(B) au moins un oxydant.

13. Utilisation selon la revendication 12, **caractérisée en ce que** l'oxydant est choisi parmi le bromate de potassium, le bromate de sodium, le perborate de sodium, l'acide déshydroascorbique, le peroxyde d'hydrogène et le peroxyde d'urée.

14. Utilisation selon la revendication 10, dans une composition de teinture pour cheveux contenant
(A) de la zéine native et
(B) un ou plusieurs précurseurs de colorants d'oxydation.
